# FASCICULE DE BREVET EUROPEEN

(11) **EP 3 865 165 B1**
(45) Date de publication et mention de la délivrance du brevet: **09.11.2022**
(21) Numéro de dépôt: 21151448.4
(22) Date de dépôt: 13.01.2021
(51) Int. Cl.: A61M 16/01, A61M 16/12

(54) **ENSEMBLE DE FOURNITURE DE GAZ AVEC SYSTÈME DE MESURE DE LA DURÉE EFFECTIVE D'UNE THÉRAPIE INHALÉE**
EINHEIT ZUR GASZUFÜHRUNG MIT SYSTEM ZUR MESSUNG DER EFFEKTIVEN DAUER EINER INHALATIONSTHERAPIE
ASSEMBLY FOR SUPPLYING GAS WITH SYSTEM FOR MEASURING THE EFFECTIVE DURATION OF AN INHALED TREATMENT

(30) Priorité: 11.02.2020 FR 2001353
(43) Date de publication de la demande: 18.08.2021
(73) Titulaire: L'AIR LIQUIDE, SOCIETE ANONYME POUR L'ETUDE ET L'EXPLOITATION DES PROCEDES GEORGES CLAUDE, 75007 Paris (FR)
(72) Inventeur: BOULANGER, Thierry, Newark, Delaware 19702 (US)
(74) Mandataire: Air Liquide

(56) Documents cités:
- EP-A1- 3 556 417
- EP-A1- 3 574 959
- EP-A2- 0 634 186
- EP-A2- 2 005 987
- EP-B1- 3 556 417

## Description

La présente invention concerne un ensemble de fourniture de gaz permettant de mesurer de la durée effective d'une thérapie inhalée, en particulier une inhalation d'un mélange gazeux N₂O/O₂ comprenant du protoxyde d'azote (N₂O) et de l'oxygène (O₂), par exemple du MEOPA, ainsi qu'une installation de fourniture de gaz comprenant une source de gaz alimentant un tel ensemble.

Le protoxyde d'azote ou N₂O est un gaz thérapeutique couramment utilisé pour traiter des patients du fait de ses propriétés analgésiques (i.e. réduction de la douleur) et anxiolytiques (i.e. réduction du stress). De plus, le N₂O ne présente pas de toxicité et conduit à des effets secondaires mineurs.

Le N₂O est généralement administré aux patients, via un masque respiratoire ou analogue, à concentration élevée, typiquement à une concentration de 50% à 70% (% molaire), le reste étant de l'oxygène (O₂). Ainsi, le mélange équimolaire (i.e. 50 mol.%/50 mol.%) de protoxyde d'azote (N₂O) et d'oxygène (O₂), appelé MEOPA, est courant utilisé pour soulager des douleurs aigues (e .g. travail de la femme enceinte, soins dentaires, petites interventions aux urgences....), et est alors inhalé de façon ponctuelle, c'est-à-dire lors des épisodes de douleur.

Par ailleurs, le N₂O peut aussi être utilisé dans le traitement de la douleur chronique, par exemple des neuropathies périphériques, exigeant une inhalation continue et sur une longue durée de N₂O, en général pendant 1 heure ou plus.

Or, il s'avère que certains patients ayant des douleurs chroniques font l'expérience d'effets secondaires mineurs du fait de l'inhalation du N₂O, en particulier un état nauséeux, et enlèvent alors leur masque en attendant que la nausée ou tout autre effet secondaire disparaisse, avant de reprendre le traitement ensuite.

De façon analogue, certains patients enlèvent leur masque pour parler à quelqu'un. Toutefois, la durée d'inhalation est jugée suffisante d'un point de vue thérapeutique, dès lors que le patient a effectivement inhalé du N₂O pendant une durée de 55 minutes par heure d'administration, c'est-à-dire qu'il est toléré que le patient retire son masque pendant au maximum 5 minutes par heure.

Or, évaluer la durée effective de traitement d'un patient n'est pas aisée lorsque le patient est traité hors d'une structure hospitalière, typiquement à son domicile, c'est-à-dire en l'absence de personnel soignant.

On connait par ailleurs EP-A-3556417 qui enseigne un système de fourniture d'un mélange O₂/N₂O donné obtenu à partir d'un prémélange dilué avec de l'air dans une chambre de mélange. Le système comprend des électrovannes proportionnelles pilotées servant à contrôler les débits de prémélange et d'air alimentant la chambre de mélange. L'administration de gaz est conforme à une prescription médicale, par exemple de 1 heure, c'est-à-dire qu'elle se stoppe automatiquement après cette durée. Ce système ne permet donc pas de savoir si le patient à bien inhalé le gaz pendant toute la durée de prescription ou interrompu l'inhalation à certains moments, par exemple enlevé son masque, comme susmentionné.

De là, le problème est de pouvoir évaluer efficacement la durée pendant laquelle un patient, en particulier traité hors structure hospitalière, typiquement à son domicile, a effectivement inhalé du N₂O, c'est-à-dire, dit autrement, de pouvoir déterminer si le patient a enlevé son masque et pendant combien de temps.

Une solution selon l'invention concerne alors un ensemble de fourniture de gaz comprenant un masque respiratoire, une valve à la demande, et un conduit de gaz reliant fluidiquement le masque respiratoire à la valve à la demande, caractérisé en ce qu'il comprend en outre :
- des moyens de traitement de signal,
- un capteur de débit ou de pression agencé dans le conduit de gaz et relié électriquement aux moyens de traitement de signal,
- un moyen d'activation comprenant un organe actionnable par un utilisateur coopérant avec les moyens de traitement de signal pour déclencher un compteur temporel principal configuré pour comptabiliser une durée principale, après actionnement par un utilisateur dudit moyen d'activation, et
- des moyens d'affichage coopérant avec moyens de traitement de signal,
et dans lequel les moyens de traitement de signal sont en outre configurés pour :
- démarrer un compteur temporel secondaire servant à comptabiliser une durée secondaire, après détection d'un début de phase expiratoire chez le patient, et
- stopper le compteur temporel principal lorsque la durée secondaire mesurée par le compteur temporel secondaire excède une valeur-seuil mémorisée.

Selon le mode de réalisation considéré, l'ensemble de fourniture de gaz selon l'invention peut comprendre l'une ou plusieurs des caractéristiques suivantes :
- la valve à la demande est configurée pour fournir du gaz de façon proportionnelle à un niveau de dépression donné (i.e. un seuil de dépression donné), c'est-à-dire qu'elle ne laisse passer le gaz que lorsque que la dépression provoquée par l'inspiration du patient atteint un niveau de dépression minimum, i.e. le seuil de dépression donné.
- la valve à la demande comprend un port de sortie.
- le seuil de dépression permettant l'ouverture de la valve à la demande est inférieur à -0.5mb.
- le port de sortie de la valve à la demande alimente le conduit de gaz reliant fluidiquement le masque respiratoire à la valve à la demande.
- la valve à la demande est configurée pour fournir du gaz de façon proportionnelle au niveau de dépression donné s'exerçant (i.e. considéré) au niveau dudit port de sortie, en particulier dans le conduit de gaz en communication fluidique avec le port de sortie.
- le capteur est préférentiellement un capteur de débit.
- le capteur de débit est avantageusement un capteur de débit massique.
- le capteur de pression est avantageusement piézorésistif.
- la valeur-seuil mémorisée est d'au moins 4 secondes, de préférence d'au moins 5 secondes, de préférence encore d'au moins 7 secondes.
- les moyens de traitement de signal sont en outre configurés pour déterminer un début de phase inspiratoire (T1) lorsque la dépression mesurée devient inférieure à environ -1mb ou lorsque que le débit mesuré excède environ 1 L/min.
- les moyens de traitement de signal sont en outre configurés pour déterminer une fin de phase inspiratoire (T1) ou un début de phase expiratoire (T2) lorsque la dépression mesurée excède environ -1mb ou lorsque le débit devient inférieur à 1 L/min.
- les moyens de traitement de signal comprennent (au moins) un microprocesseur, de préférence un microcontrôleur.
- les moyens de traitement de signal comprennent une carte électronique portant ledit au moins un microprocesseur.
- ledit au moins un microprocesseur met en œuvre au moins un algorithme
- il comprend un boitier dans lequel sont agencés au moins les moyens de traitement de signal, le moyen d'activation actionnable par l'utilisateur et les moyens d'affichage.
- le capteur de débit ou de pression est configuré pour :
   i) mesurer au moins un débit ou une pression de gaz au sein du conduit, et
   ii) délivrer au moins un signal représentatif dudit au moins un débit de gaz ou de ladite au moins une pression mesuré aux moyens de traitement de signal.
- les moyens de traitement de signal sont configurés pour recueillir et traiter les signaux de débit ou de pression de gaz mesurés par le capteur de débit ou de pression.
- les moyens de traitement de signal sont configurés pour recueillir et traiter les signaux de débit ou de pression de gaz mesurés par le capteur de débit ou de pression après activation par l'utilisateur du moyen d'activation.
- le moyen d'activation comprend un organe d'activation choisi parmi les touches, les boutons-poussoirs ou tout autre dispositif analogue.
- les moyens de traitement de signal sont en outre configurés pour démarrer un compteur temporel secondaire servant à comptabiliser une durée secondaire, après détection d'un début de phase expiratoire (T2) chez le patient.
- les moyens de traitement de signal sont en outre configurés pour stopper le compteur temporel principal lorsque la durée secondaire mesurée par le compteur temporel secondaire excède à un instant donné une valeur-seuil mémorisée par les moyens de traitement de signal.
- les moyens de traitement de signal sont en outre configurés pour redémarrer le compteur temporel principal et réinitialiser le compteur temporel secondaire (i.e. remise à zéro), après détection d'un début de phase inspiratoire (T1) chez le patient.
- les moyens de traitement de signal sont configurés pour détecter un début de phase expiratoire (T2) ou un début de phase inspiratoire (T1) chez le patient à partir du débit ou de la pression de gaz mesuré par le capteur de débit ou de pression.
- il comprend en outre des moyens d'affichage, tel un afficheur (i.e. écran d'affichage) configurés pour afficher la durée principale, par exemple un écran type LCD ou autre.
- il comprend en outre une source de gaz reliée fluidiquement à la valve à la demande, en particulier la source de gaz comprend une bouteille de gaz contenant un mélange N₂O/O₂.
- il est préférentiellement un masque nasal, c'est-à-dire destiné à recouvrir la région nasale de l'utilisateur.
- le masque comprend une ouverture large en communication fluidique avec la chambre respiratoire recevant au moins une partie du nez d'un utilisateur, pendant l'utilisation du masque. L'ouverture large est préférentiellement bordée par un coussin flexible assurant un confort d'utilisation et une étanchéité gazeuse. Le coussin flexible est par exemple en polymère, par exemple en silicone, et il est fixé au rebord délimitant l'ouverture.
- le boitier comprend en outre une interface utilisateur comprenant le moyen d'activation actionnable par un utilisateur et les moyens d'affichage.
- il comprend des moyens d'alimentation électrique, notamment (au moins) une batterie, pile ou liaison de raccordement au secteur.
- les moyens d'alimentation électrique alimentent au moins les moyens de traitement de signal et le capteur de débit ou de pression.

L'invention va maintenant être mieux comprise grâce à la description détaillée suivante, faite à titre illustratif mais non limitatif, en référence aux figures annexées parmi lesquelles :
Fig. 1 schématise un ensemble d'administration d'un mélange gazeux N₂O/O₂ classique,
Fig. 2 schématise l'utilisation par un patient d'un ensemble d'administration de gaz selon l'invention,
Fig. 3 illustre un arrêt momentané de l'utilisation par le patient de l'ensemble d'administration de gaz de la Fig. 2,
Fig. 4 est une courbe illustrant les données de mesure fournies par le capteur de débit de l'ensemble d'administration de gaz des Fig. 2 et Fig. 3, et
Fig. 5 est une courbe illustrant les données de mesure fournies par le capteur de pression de l'ensemble d'administration de gaz des Fig. 2 et Fig. 3.

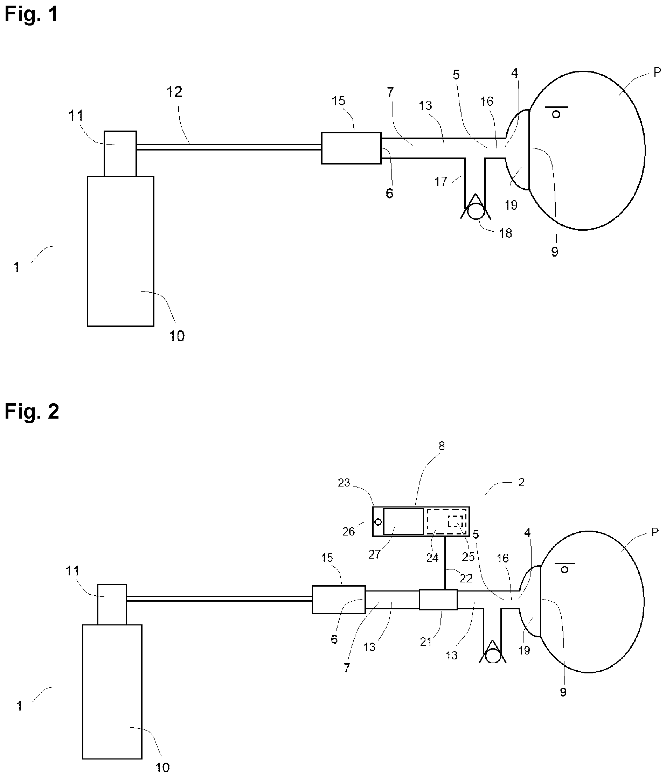

Fig. 1 schématise un ensemble d'administration classique d'un mélange gazeux N₂O/O₂ (appelé « gaz » ci-après) contenant du N₂O et de l'oxygène (O₂), à savoir ici du MEOPA (i.e. 50%/50% molaire de N₂O et O₂) à un patient P.

Le gaz provient d'une source de gaz 1, à savoir ici une bouteille de gaz 10 dans laquelle le gaz est stocké sous pression, typiquement à plus de 150 bar abs, par exemple à environ 170 bars. La bouteille 10 est équipée d'un détendeur 11 permettant de réaliser une détente du gaz, c'est-à-dire une réduction de sa pression, par exemple de 150 bar à 5 bar abs. Le détendeur 11 est typiquement connecté à un dispositif d'administration de gaz du type valve à la demande 15, via un tuyau flexible 12 ou analogue, véhiculant le gaz. La valve à la demande 15 délivre le gaz dans la partie amont 7 d'un conduit de gaz 13, de façon proportionnelle à un niveau de dépression présent à son port de sortie 6. La valve à la demande 15 est par exemple celle commercialisée sous la référence Ease II^{™} par la société GCE.

Le patient P est alimenté en gaz par le conduit de gaz 13 et un masque respiratoire 19 de type nasal ou facial (i.e. bucco-nasal), alimenté en gaz par la valve à la demande 15 et le conduit de gaz 13. Le masque respiratoire 19 vient recouvrir la région nasale ou les régions nasale et buccale de l'utilisateur, i.e. le patient P. De tels masques respiratoires 19 sont bien connus et décrits par exemple par EP-A-3574959, EP-A-2005987, EP-A-634186...

Typiquement, un tel masque 19 comprend un corps de masque définissant une chambre. Le masque 19 est positionné sur la région nasale ou bucco-nasale du patient P, pendant son utilisation normale, c'est-à-dire lorsqu'il y inhale et y exhale du gaz, de préférence un masque nasal venant recouvrir la région nasale de l'utilisateur.

Un orifice de passage de gaz 4, i.e. d'entrée et sortie de gaz, est agencé au travers du corps de masque et en communication fluidique avec la chambre respiratoire pour introduire du gaz, i.e. mélange N₂O/O₂ par exemple, ou extraire du gaz de ladite chambre respiratoire, en particulier les gaz riches en CO₂ expirés par le patient P, pendant ses phases expiratoires. L'orifice de passage de gaz 4 du masque 19 communique fluidiquement avec la portion aval 5 du conduit de gaz 13 de manière à ce que le gaz à inhaler puisse être fourni par le conduit de gaz 13 au masque 19 pendant les phases inspiratoires du patient P.

Le masque 19 comprend aussi une ouverture large 9, en général bordée par un coussin souple (non schématisé), par exemple en silicone ou en élastomère, fixé à la bordure ou pourtour périphérique bordant l'ouverture large de la chambre respiratoire. Le coussin et l'ouverture large sont dimensionnés pour accueillir, au moins une partie du nez de l'utilisateur, pendant l'utilisation du masque 19, de manière à ce que celui-ci puisse respirer le gaz contenu dans la chambre respiratoire du masque 19, c'est-à-dire inhaler du gaz thérapeutique qui s'y trouve pendant ses phases inspiratoires et y exhaler du gaz riche en CO₂, pendant ses phases expiratoires, lequel est ensuite évacué vers l'atmosphère via un conduit expiratoire 17.

Pendant l'utilisation du masque 19, lorsque le patient P inspire, c'est-à-dire pendant ses phases inspiratoires, une dépression apparaît dans la chambre respiratoire du corps de masque puis dans le conduit 13 qui relie fluidiquement le masque 19 au port de sortie 6 de la valve à la demande 15.

Il est prévu en outre un conduit expiratoire 17, aussi appelé conduit d'échappement, dans lequel est agencée une valve unidirectionnelle 18 communiquant avec l'atmosphère ambiante, lequel conduit expiratoire 17 est connecté fluidiquement au conduit de gaz 13 dans sa région aval 5.

En réponse à la dépression due à l'inspiration du patient, qui se crée dans la portion de conduit 16 situé dans la partie aval 5 du conduit 13, la valve unidirectionnelle 18 se ferme pour empêcher l'air ambiant d'entrer dans le conduit expiratoire 17, ce qui permet à la dépression de se propager vers la partie amont 7 du conduit inspiratoire 13 jusqu'à la valve à la demande 15, engendrant alors une ouverture du port de sortie 6 de la valve à la demande 15, laquelle laisse alors passer du gaz qui peut circuler dans le conduit de gaz 13 depuis sa portion amont 7 à sa portion aval 5. Le gaz pénètre alors dans le masque 19, via l'orifice 4, et est alors fourni au patient P. Plus le niveau de dépression est élevé, traduisant l'intensité de la demande du patient P, plus le débit délivré par la valve à la demande 15 et fourni au patient P est important.

A l'inverse, lors des phases expiratoires, les gaz riches en CO₂ expirés par le patient P dans la chambre respiratoire du corps de masque, en ressort par l'orifice de passage de gaz 4, circulent au travers de la portion aval 5 du conduit 13, puis s'échappent à l'air ambiant via le conduit expiratoire 17 et la valve unidirectionnelle 18. La valve à la demande 15 s'oppose quant à elle à toute circulation des gaz expirés dans le conduit inspiratoire 13, c'est-à-dire à toute remontée de gaz en direction de la source de gaz 1.

Avec un tel système d'administration de gaz, il est toutefois impossible de connaitre la durée pendant laquelle le patient P a enlevé son masque respiratoire 19, par exemple en attendant qu'un éventuel état nauséeux disparaisse, pour parler au téléphone ou toute autre raison.

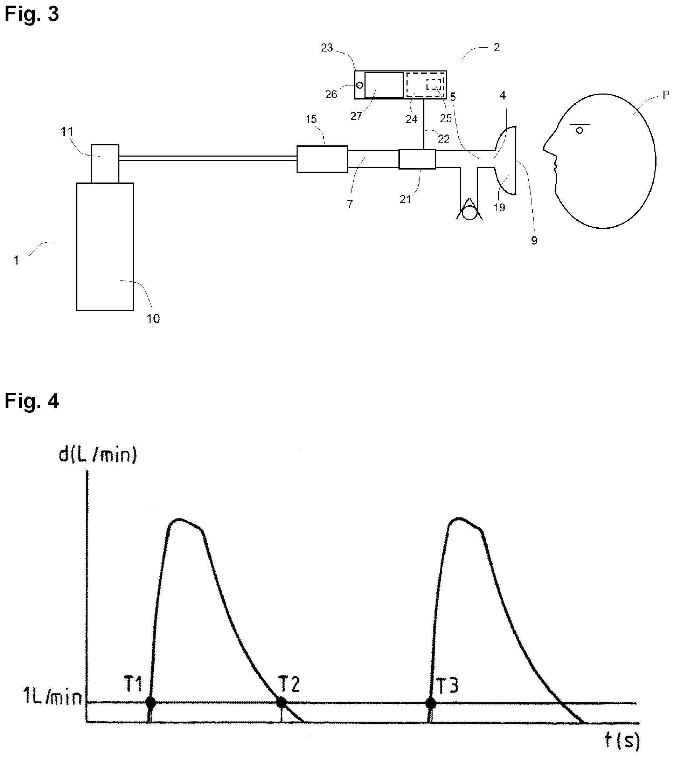

En effet, alors que la plupart des patients P supporte aisément une thérapie d'une heure, certains peuvent avoir de brèves périodes d'état nauséeux pendant lesquelles le masque 19 est retiré afin de respirer l'air ambient et attendre que l'état nauséeux s'estompe, comme illustré en Fig. 3.

Pour résoudre ce problème, selon l'invention, l'installation de la Fig.1 a été modifiée par ajout d'un système de suivi 2 comprenant des moyens de mesure, typiquement un capteur de débit ou de pression 21, coopérant avec des moyens de traitement de signal 8, comme illustré sur les Fig. 2 et Fig. 3, de préférence un capteur de débit 21.

Plus précisément, dans le mode de réalisation illustré sur les Figures et détaillé ci-après, on utilise comme capteur, un capteur de débit 21 qui est inséré dans le conduit de gaz 13, c'est-à-dire entre le masque 19 et la valve 15, lequel capteur de débit 21 est par ailleurs relié électriquement à des moyens de traitement de signal 8. Ce capteur de débit 21 est configuré et agencé de manière à opérer des mesures de débit dans le conduit de gaz 13.

Autrement dit, selon l'invention, il est proposé un ensemble de fourniture de gaz 2 comprenant un masque respiratoire 19, une valve à la demande 15, un conduit de gaz 13 reliant fluidiquement le masque respiratoire 19 à la valve à la demande 15, des moyens de traitement de signal 8 et un capteur de débit 21, agencé dans le conduit de gaz 13, lequel est relié électriquement aux moyens de traitement de signal 8.

De préférence, le capteur de débit 21 est agencé sur ou dans la paroi interne conduit de gaz 13 ou porté par un élément de conduit indépendant venant se raccorder fluidiquement au conduit de gaz 13.

Le capteur de débit 21 est connecté électriquement, via un câble électrique 22, aux moyens de traitement de signal 8, lesquels comprennent préférentiellement une carte électronique 24 permettant d'alimenter électriquement et recueillir et traiter les signaux, i.e. données de mesure, provenant du capteur 21.

La carte électronique 24 est insérée dans un châssis ou boitier 23 comprenant en outre une interface utilisateur 26, 27 incluant un moyen d'activation 26 actionnable par le patient P, par exemple un bouton poussoir, ainsi que des moyens d'affichage 27, tel un afficheur d'informations.

Le capteur de débit 21 est alimenté électriquement par la carte électronique 24. De même, le moyen d'activation 26 et les moyens d'affichage 27 sont aussi alimentés électriquement par la carte électronique 24 par exemple par l'intermédiaire d'un jeu de torons (non montrés). L'alimentation électrique peut être réalisée de multiples manières, par exemple au moyen d'une alimentation externe, une batterie etc...

La carte électronique 24 intègre un microcontrôleur 25 réalisant les différentes opérations et traitements par le biais d'un ou plusieurs algorithmes, afin d'exploiter, i.e. de traiter informatiquement, les signaux de mesure provenant du capteur de débit 21, et par ailleurs de restituer ensuite des données sur l'afficheur 27, tel un écran LCD ou analogue, comme détaillé ci-après.

Comme illustré en Fig. 2, une fois que le patient a mis en place son masque 19 pour commencer les inhalations/expirations et appuyé sur le bouton poussoir 26, le microcontrôleur 25 des moyens de traitement de signal 8 commence à recueillir et analyser les signaux transmis par le capteur de débit 21.

Préférentiellement, le capteur de débit 21 est un capteur de débit massique, c'est-à-dire un capteur de débit dont la mesure se fait à partir de la masse du gaz et non de son volume. Par exemple, on peut utiliser le capteur référencé SFM3000 de la société Sensirion ou tout autre capteur similaire.

Le fonctionnement du système de suivi 2 comprenant le capteur de débit 21 et les moyens de traitement de signal 8 est le suivant.

Lorsque le patient a placé son masque 19 pour commencer les inhalations et expirations de gaz, comme illustré en Fig. 2, il active le système 2 en appuyant sur le bouton poussoir 26, ce qui donne l'ordre au microcontrôleur 25 de déclencher un compteur temporel principal servant à comptabiliser une durée principale, en particulier la durée effective de traitement du patient et de commencer à recueillir et analyser les données, à savoir le débit (d) de gaz, par exemple exprimé en L/min. Les signaux de mesure sont transmis par le capteur de débit 21 au microcontrôleur 25 et rafraichies par exemple toutes les 10 ms.

Les données issues du capteur de débit 21 sont illustrées sur la Fig. 4.

Les inhalations de gaz par le patient correspondent à des valeurs de débit strictement positives, caractérisant le débit délivré par la valve à la demande 15, alors que les expirations à des valeurs nulles considérant que la valve à la demande 15 force l'intégralité du gaz expiré par le patient d'emprunter le conduit expiratoire 17.

Le microcontrôleur 25 peut déterminer que le patient inhale lorsque que le débit (d) est supérieur à 1 L/min et, à l'inverse, exhale lorsque que le débit est inférieur à 1 L/min.

L'alternance entre les phases inspiratoire et expiratoire s'opère lors de transitions T, par exemple quand le débit devient pour la première fois, supérieur à 1 L/min (i.e. temps T1 et T3), ce qui correspond au début de l'inhalation, i.e. un début de phase inspiratoire du patient P, ou à l'inverse lorsque le débit devient pour la première fois, inférieur à 1 L/min (i.e. temps T2), ce qui correspond au début de l'expiration, i.e. un début de phase expiratoire du patient P.

En ayant déterminé ces phases transitoires, le microcontrôleur 25 est en capacité notamment de mesurer le temps d'une expiration, qui correspond au segment (T2-T3) de la Fig. 4.

Alors que la plupart des patients supportent aisément une thérapie d'une heure, certains peuvent avoir de brèves périodes d'état nauséeux pendant lesquelles ils retirent leur masque 19 afin de respirer l'air ambient et attendre que l'état nauséeux s'estompe (cf. Fig. 3).

Dans une telle situation (i.e. retrait du masque), le débit mesuré par le capteur de débit 21 est nul, donc inférieur à 1 L/min, et donc est considéré comme une expiration par le microcontrôleur 25, c'est-à-dire une phase expiratoire.

Il est à noter que :
- si le patient retire le masque alors qu'il est en phase expiratoire, alors la transition T2 a déjà été déterminée ;
- si le patient retire le masque en pleine inhalation alors le débit passe brutalement d'une valeur supérieure à 1 L/min à une valeur inférieure à 1 L/min, permettant alors au microcontrôleur 25 de déterminer la transition T2.

Dans tous les cas, après détermination de la transition T2, le microcontrôleur 25 active en parallèle du compteur temporel principal servant à comptabiliser une durée principale, un compteur temporel secondaire servant à comptabiliser une durée secondaire.

Le patient P ayant retiré le masque 19, ce compteur temporel secondaire va excéder à un instant donné une valeur-seuil, i.e. une durée donnée, mémorisée dans la carte électronique 24 à laquelle vient se comparer le microcontrôleur 25. La valeur-seuil peut par exemple être de l'ordre de 5 à 7 secondes ou une autre durée fixée.

A partir du moment où le compteur temporel secondaire excède la valeur-seuil préfixée, i.e. mémorisée, le microcontrôleur stoppe le compteur temporel principal, signalant ainsi que le patient P a retiré le masque 19.

Lorsque le patient remet son masque 19, une transition de type T1, c'est-à-dire un passage (i.e. début) en phase inspiratoire, est détectée ce qui permet au microcontrôleur 25 de redémarrer le compteur temporel principal (à la valeur précédemment atteinte) et de réinitialiser le compteur temporel secondaire à 0.

La thérapie voit donc se succéder pour certains patients, une alternance d'adhérence à la thérapie et de pauses. Selon l'invention, le microcontrôleur 25 est en capacité de mesurer la durée effective du traitement en déterminant les phases où le patient P a retiré son masque et de stopper alors le compteur temporel principal.

Autrement dit, la durée principale comptabilisée par le compteur principal correspond à la durée effective du traitement puisque le temps de non-port du masque n'est pas comptabilisé, c'est-à-dire le temps pendant lequel le compteur temporel secondaire est mis en route.

Après une durée d'administration donnée, par exemple après 1 heure d'administration, le patient P arrête la thérapie et retire son masque 19. En consultant l'afficheur 27 du boitier 23, qui restitue la durée effective de traitement fournie par les moyens de traitement 8, typiquement le microcontrôleur 25, il est alors possible de savoir si le patient a inhalé le gaz pendant la durée minimum requise, par exemple 55 minutes.

Le système 2 de l'invention peut intégrer des éléments supplémentaires pour faciliter l'administration de la thérapie. Par exemple, le microcontrôleur 25 peut aussi déclencher en parallèle un décompte temporel fixé à 1h dès lors que l'utilisateur appuie sur le bouton poussoir 26.

De même, il peut déclencher une alerte du type signal auditif et/ou visuel servant à indiquer que l'administration a atteint 1h, indépendamment des éventuelles pauses réalisées par le patient P.

Autrement dit, le microcontrôleur 25 est en capacité de mesurer la durée effective du traitement, en cumulant le temps uniquement lorsque le patient P inhale le gaz thérapeutique, après 1 heure d'administration par exemple.

Le mode de réalisation présenté ci-dessus met en œuvre un capteur de débit mais on peut aussi utiliser, selon un autre mode de réalisation, un capteur de pression à la place du capteur de débit.

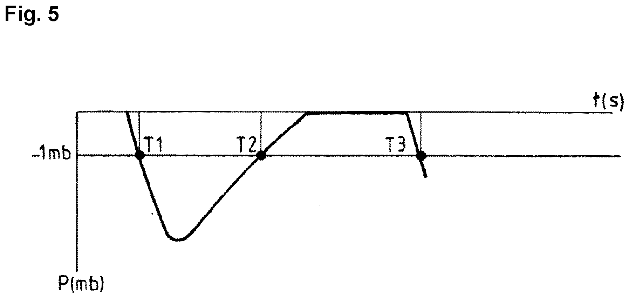

Ceci est illustré par la Fig. 5 où les transitions T sont déterminées par le microcontrôleur 25 à partir d'un seuil de pression négative. En effet, lorsque le patient P initie une inhalation, la valve à la demande 15 génère une résistance proportionnelle à la demande du patient P, se traduisant par une pression négative (i.e. une dépression) dans le conduit de gaz 13.

Ainsi, le début d'une inhalation peut être déterminé dès lors que la pression mesurée par le capteur de pression 21 (utilisé à la place du capteur de débit) devient inférieure à par exemple environ -1mb, et la fin de l'inhalation déterminée dès lors que la pression redevient supérieure à par exemple environ -1mb.

Comme décrit précédemment, le microcontrôleur 25 est en mesure de précisément déterminer les transitions T (T1, T2, T3...) sur la base des mesures fournies par le capteur de pression 21 et en déduire la durée effective du traitement, en cumulant le temps uniquement lorsque le patient P inhale le gaz thérapeutique, après 1 heure d'administration par exemple.

Dans tous les cas, qu'il fonction avec un capteur de débit ou un capteur de pression, l'ensemble de fourniture de gaz 2 de l'invention permet de déterminer efficacement la durée pendant laquelle un patient, en particulier traité hors structure hospitalière, typiquement à son domicile, a effectivement inhalé du N₂O et/ou, à l'inverse, a enlevé son masque et pendant combien de temps.

## Revendications

1. Ensemble de fourniture de gaz (2) comprenant :
- un masque respiratoire (19),
- une valve à la demande (15), et
- un conduit de gaz (13) reliant fluidiquement le masque respiratoire (19) à la valve à la demande (15),
**caractérisé en ce qu'**il comprend en outre :
- des moyens de traitement de signal (8),
- un capteur de débit ou de pression (21) agencé dans le conduit de gaz (13) et relié électriquement aux moyens de traitement de signal (8),
- un moyen d'activation (26) comprenant un organe actionnable par un utilisateur coopérant avec les moyens de traitement de signal (8) pour déclencher un compteur temporel principal configuré pour comptabiliser une durée principale, après actionnement par un utilisateur dudit moyen d'activation (26), et
- des moyens d'affichage (27) coopérant avec moyens de traitement de signal (8),
et dans lequel les moyens de traitement de signal (8) sont en outre configurés pour :
a) démarrer un compteur temporel secondaire servant à comptabiliser une durée secondaire, après détection d'un début de phase expiratoire (T2) chez le patient, et
b) stopper le compteur temporel principal lorsque la durée secondaire mesurée par le compteur temporel secondaire excède une valeur-seuil mémorisée.

2. Ensemble selon la revendication précédente, **caractérisé en ce que** le capteur est un capteur de débit massique.

3. Ensemble selon l'une des revendications précédentes, **caractérisé en ce que** les moyens de traitement de signal (8) comprennent un microprocesseur (25), de préférence un microcontrôleur.

4. Ensemble selon l'une des revendications précédentes, **caractérisé en ce qu'**il comprend un boitier (23) dans lequel sont agencés au moins les moyens de traitement de signal (8), le moyen d'activation (26) actionnable par l'utilisateur et les moyens d'affichage (27).

5. Ensemble selon l'une des revendications précédentes, **caractérisé en ce que** le capteur de débit ou de pression (21) est configuré pour :
- mesurer au moins un débit ou une pression de gaz au sein du conduit (13), et
- délivrer au moins un signal représentatif dudit au moins un débit de gaz ou de ladite au moins une pression de gaz mesuré aux moyens de traitement de signal (8).

6. Ensemble selon l'une des revendications précédentes, **caractérisé en ce que** les moyens de traitement de signal (8) sont configurés pour recueillir et traiter les signaux de débit de gaz ou de pression mesurés par le capteur de débit ou de pression (21).

7. Ensemble selon l'une des revendications précédentes, **caractérisé en ce que** les moyens de traitement de signal (8) sont configurés pour détecter un début de phase expiratoire (T2) ou un début de phase inspiratoire (T1) chez le patient à partir du débit ou de la pression de gaz mesuré par le capteur de débit ou de pression (21).

8. Ensemble selon l'une des revendications précédentes, **caractérisé en ce que** les moyens de traitement de signal (8) sont en outre configurés pour démarrer le compteur temporel secondaire servant à comptabiliser la durée secondaire, après détection d'un début de phase expiratoire (T2) chez le patient.

9. Ensemble selon l'une des revendications précédentes, **caractérisé en ce que** la valeur-seuil est mémorisée par les moyens de traitement de signal (8).

10. Ensemble selon l'une des revendications précédentes, **caractérisé en ce que** la valeur-seuil mémorisée est d'au moins 4 secondes, de préférence d'au moins 5 secondes, de préférence encore d'au moins 7 secondes.

11. Ensemble selon l'une des revendications précédentes, **caractérisé en ce que** les moyens de traitement de signal (8) sont en outre configurés pour redémarrer le compteur temporel principal et réinitialiser le compteur temporel secondaire (i.e. remise à zéro), après détection d'un début de phase inspiratoire (T1) chez le patient.

12. Ensemble selon l'une des revendications précédentes, **caractérisé en ce que** les moyens de traitement de signal (8) sont en outre configurés pour déterminer un début de phase inspiratoire (T1) lorsque la dépression mesurée devient inférieure à environ -1mb ou lorsque que le débit mesuré excède environ 1 L/min.

13. Ensemble selon l'une des revendications précédentes, **caractérisé en ce que** les moyens de traitement de signal (8) sont en outre configurés pour déterminer une fin de phase inspiratoire (T1) ou un début de phase expiratoire (T2) lorsque la dépression mesurée excède environ -1mb ou lorsque le débit devient inférieur à 1 L/min.

14. Ensemble selon l'une des revendications précédentes, caractérisé en ce le moyen d'activation comprend un organe d'activation choisi parmi les touches, les boutons-poussoirs.

15. Ensemble selon l'une des revendications précédentes, **caractérisé en ce qu'**il comprend en outre des moyens d'affichage (27), tel un afficheur (i.e. écran d'affichage) configuré pour afficher la durée principale.

16. Ensemble selon l'une des revendications précédentes, **caractérisé en ce qu'**il comprend en outre une source de gaz (1) reliée fluidiquement à la valve à la demande (15), en particulier la source de gaz (1) comprend une bouteille de gaz contenant un mélange N₂O/O₂.

## Patentansprüche

1. Anordnung zur Zuführung von Gas (2), umfassend:
- eine Atemmaske (19),
- ein Demandventil (15) und
- eine Gasleitung (13), die die Atemmaske (19) fluidisch mit dem Demandventil (15) verbindet,
**dadurch gekennzeichnet, dass** sie ferner umfasst:
- Signalverarbeitungsmittel (8),
- einen Durchfluss- oder Drucksensor (21), der in der Gasleitung (13) angeordnet ist und mit den Signalverarbeitungsmitteln (8) elektrisch verbunden ist,
- ein Aktivierungsmittel (26), das ein von einem Benutzer betätigbares Element umfasst, das mit den Signalverarbeitungsmitteln (8) zusammenwirkt, um einen Hauptzeitzähler auszulösen, der dazu ausgestaltet ist, nach Betätigung des Aktivierungsmittels (26) durch einen Benutzer eine Hauptdauer zu erfassen, und
- Anzeigemittel (27), die mit den Signalverarbeitungsmitteln (8) zusammenwirken,
und wobei die Signalverarbeitungsmittel (8) ferner dazu ausgestaltet sind:
a) einen sekundären Zeitzähler zu starten, der dazu dient, nach Detektion eines Beginns einer Ausatemphase (T2) bei dem Patienten eine sekundäre Dauer zu erfassen, und
b) den Hauptzeitzähler zu stoppen, wenn die von dem sekundären Zeitzähler gemessene sekundäre Dauer einen gespeicherten Schwellenwert überschreitet.

2. Anordnung nach dem vorhergehenden Anspruch, **dadurch gekennzeichnet, dass** der Sensor ein Massendurchflusssensor ist.

3. Anordnung nach einem der vorhergehenden Ansprüche, **dadurch gekennzeichnet, dass** die Signalverarbeitungsmittel (8) einen Mikroprozessor (25) umfassen, bevorzugt einen Mikrocontroller.

4. Anordnung nach einem der vorhergehenden Ansprüche, **dadurch gekennzeichnet, dass** sie ein Gehäuse (23) umfasst, in dem mindestens die Signalverarbeitungsmittel (8), das von dem Benutzer betätigbare Aktivierungsmittel (26) und die Anzeigemittel (27) angeordnet sind.

5. Anordnung nach einem der vorhergehenden Ansprüche, **dadurch gekennzeichnet, dass** der Durchfluss- oder Drucksensor (21) dazu ausgestaltet ist:
- mindestens einen Gasdurchfluss oder -druck in der Leitung (13) zu messen und
- mindestens ein Signal, das für den mindestens einen gemessenen Gasdurchfluss oder den mindestens einen gemessenen Gasdruck repräsentativ ist, an die Signalverarbeitungsmittel (8) abzugeben.

6. Anordnung nach einem der vorhergehenden Ansprüche, **dadurch gekennzeichnet, dass** die Signalverarbeitungsmittel (8) dazu ausgestaltet sind, die von dem Durchfluss- oder Drucksensor (21) gemessenen Gasdurchfluss- oder Drucksignale aufzunehmen und zu verarbeiten.

7. Anordnung nach einem der vorhergehenden Ansprüche, **dadurch gekennzeichnet, dass** die Signalverarbeitungsmittel (8) dazu ausgestaltet sind, einen Beginn einer Ausatemphase (T2) oder einen Beginn einer Einatemphase (T1) bei dem Patienten anhand des von dem Durchfluss- oder Drucksensor (21) gemessenen Gasdurchflusses oder -drucks zu detektieren.

8. Anordnung nach einem der vorhergehenden Ansprüche, **dadurch gekennzeichnet, dass** die Signalverarbeitungsmittel (8) ferner dazu ausgestaltet sind, den sekundären Zeitzähler, der zum Erfassen der sekundären Dauer dient, nach Detektion eines Beginns einer Ausatemphase (T2) bei dem Patienten zu starten.

9. Anordnung nach einem der vorhergehenden Ansprüche, **dadurch gekennzeichnet, dass** der Schwellenwert von den Signalverarbeitungsmitteln (8) gespeichert wird.

10. Anordnung nach einem der vorhergehenden Ansprüche, **dadurch gekennzeichnet, dass** der gespeicherte Schwellenwert mindestens 4 Sekunden, bevorzugt mindestens 5 Sekunden, noch bevorzugter mindestens 7 Sekunden beträgt.

11. Anordnung nach einem der vorhergehenden Ansprüche, **dadurch gekennzeichnet, dass** die Signalverarbeitungsmittel (8) ferner dazu ausgestaltet sind, nach Detektion eines Beginns einer Einatemphase (T1) bei dem Patienten den Hauptzeitzähler neu zu starten und den sekundären Zeitzähler zu reinitialisieren (d. h. Zurücksetzen auf null).

12. Anordnung nach einem der vorhergehenden Ansprüche, **dadurch gekennzeichnet, dass** die Signalverarbeitungsmittel (8) ferner dazu ausgestaltet sind, einen Beginn einer Einatemphase (T1) zu bestimmen, wenn der gemessene Unterdruck geringer als ungefähr -1mb wird oder wenn der gemessene Durchfluss ungefähr 1 L/min überschreitet.

13. Anordnung nach einem der vorhergehenden Ansprüche, **dadurch gekennzeichnet, dass** die Signalverarbeitungsmittel (8) ferner dazu ausgestaltet sind, ein Ende einer Einatemphase (T1) oder einen Beginn einer Ausatemphase (T2) zu bestimmen, wenn der gemessene Unterdruck ungefähr -1mb überschreitet oder wenn der Durchfluss geringer als 1 L/min wird.

14. Anordnung nach einem der vorhergehenden Ansprüche, **dadurch gekennzeichnet, dass** das Aktivierungsmittel ein Aktivierungselement umfasst, das unter Tasten, Drucktastern gewählt ist.

15. Anordnung nach einem der vorhergehenden Ansprüche, **dadurch gekennzeichnet, dass** sie ferner Anzeigemittel (27) umfasst wie etwa ein Display (d. h. einen Anzeigebildschirm), das dazu ausgestaltet ist, die Hauptdauer anzuzeigen.

16. Anordnung nach einem der vorhergehenden Ansprüche, **dadurch gekennzeichnet, dass** sie ferner eine Gasquelle (1) umfasst, die fluidisch mit dem Demandventil (15) verbunden ist, wobei die Gasquelle (1) im Besonderen eine Gasflasche umfasst, die ein N₂O/O₂-Gemisch enthält.

## Claims

1. Assembly (2) for supplying gas, comprising:
- a breathing mask (19),
- a demand valve (15), and
- a gas conduit (13) fluidically connecting the breathing mask (19) to the demand valve (15),
**characterized in that** it further comprises:
- signal processing means (8),
- a flow or pressure sensor (21) arranged in the gas conduit (13) and electrically connected to the signal processing means (8),
- an activation means (26) comprising a member actuatable by a user, cooperating with the signal processing means (8) in order to trigger a main time counter configured to count a main duration, after actuation of said activation means (26) by a user, and
- display means (27) cooperating with signal processing means (8),
and in which the signal processing means (8) are further configured to:
a) start a secondary time counter used for counting a secondary duration, after detection of a start of an expiratory phase (T2) in the patient, and
b) stop the main time counter when the secondary duration measured by the secondary time counter exceeds a stored threshold value.

2. Assembly according to the preceding claim, **characterized in that** the sensor is a mass flow sensor.

3. Assembly according to one of the preceding claims, **characterized in that** the signal processing means (8) comprise a microprocessor (25), preferably a microcontroller.

4. Assembly according to one of the preceding claims, **characterized in that** it comprises a casing (23) in which are arranged at least the signal processing means (8), the activation means (26) actuatable by the user, and the display means (27).

5. Assembly according to one of the preceding claims, **characterized in that** the flow or pressure sensor (21) is configured to:
- measure at least one gas flow or gas pressure within the conduit (13), and
- deliver at least one signal representative of said at least one measured gas flow or of said at least one measured gas pressure to the signal processing means (8).

6. Assembly according to one of the preceding claims, **characterized in that** the signal processing means (8) are configured to collect and process the gas flow or pressure signals measured by the flow or pressure sensor (21).

7. Assembly according to one of the preceding claims, **characterized in that** the signal processing means (8) are configured to detect a start of an expiratory phase (T2) or a start of an inspiratory phase (T1) in the patient on the basis of the gas flow or gas pressure measured by the flow or pressure sensor (21).

8. Assembly according to one of the preceding claims, **characterized in that** the signal processing means (8) are further configured to start the secondary time counter used for counting the secondary duration, after detection of a start of an expiratory phase (T2) in the patient.

9. Assembly according to one of the preceding claims, **characterized in that** the threshold value is stored by the signal processing means (8).

10. Assembly according to one of the preceding claims, **characterized in that** the stored threshold value is at least 4 seconds, preferably at least 5 seconds, more preferably at least 7 seconds.

11. Assembly according to one of the preceding claims, **characterized in that** the signal processing means (8) are further configured to restart the main time counter and reset the secondary time counter (i.e. reset to zero), after detection of a start of an inspiratory phase (T1) in the patient.

12. Assembly according to one of the preceding claims, **characterized in that** the signal processing means (8) are further configured to determine a start of an inspiratory phase (T1) when the negative pressure measured becomes less than approximately -1 mb or when the measured flow exceeds approximately 1 l/min.

13. Assembly according to one of the preceding claims, **characterized in that** the signal processing means (8) are further configured to determine an end of an inspiratory phase (T1) or a start of an expiratory phase (T2) when the negative pressure measured exceeds approximately -1 mb or when the flow becomes less than 1 l/min.

14. Assembly according to one of the preceding claims, **characterized in that** the activation means comprises an activation member chosen from keys and push buttons.

15. Assembly according to one of the preceding claims, **characterized in that** it further comprises display means (27), such as a display (i.e. display screen) configured to display the main duration.

16. Assembly according to one of the preceding claims, **characterized in that** it further comprises a gas source (1) fluidically connected to the demand valve (15), and in particular the gas source (1) comprises a gas cylinder containing an N₂O/O₂ mixture.
